(19) Europäisches Patentamt
European Patent Office
Office européen des brevets

(11) **EP 4 265 259 A1**

(12) **EUROPEAN PATENT APPLICATION**
published in accordance with Art. 153(4) EPC

(43) Date of publication:
**25.10.2023 Bulletin 2023/43**

(21) Application number: **21905870.8**

(22) Date of filing: **13.12.2021**

(51) International Patent Classification (IPC):
**A61K 31/7048** (2006.01)  **A61K 47/40** (2006.01)
**A61K 47/26** (2006.01)  **A61K 47/18** (2017.01)
**A61K 9/72** (2006.01)  **A61P 31/10** (2006.01)

(52) Cooperative Patent Classification (CPC):
Y02A 50/30

(86) International application number:
**PCT/ES2021/070885**

(87) International publication number:
**WO 2022/129661 (23.06.2022 Gazette 2022/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.12.2020 ES 202031248**

(71) Applicants:
• **Universidad Complutense De Madrid**
  **28040 Madrid (ES)**
• **Universidad Cardenal Herrera**
  **46115 Valencia (ES)**
• **Trinity College of Dublin**
  **Dublin 2 (IE)**
• **French National Institute of Health and Medical**
  **Research (Inserm)**
  **75654 Paris Cedex 13 (FR)**
• **Poitiers University Hospital**
  **86021 Poitiers (FR)**
• **Poitiers University**
  **71117 Poitiers (FR)**

(72) Inventors:
• **SERRANO LOPEZ, Dolores Remedios**
  **28040 Madrid (ES)**
• **DE PABLO TOMERO, Esther**
  **28040 Madrid (ES)**
• **TORRADO DURÁN, Juan José**
  **28040 Madrid (ES)**
• **BALLESTEROS PAPANTONAKIS, María Paloma**
  **28040 Madrid (ES)**
• **BOLÁS FERNÁNDEZ, Francisco**
  **28040 Madrid (ES)**
• **HEALY, Anne Marie**
  **DUBLIN, Dublin 2 (IE)**
• **MARCHAND, Sandrine**
  **86022 Poitiers (FR)**
• **DEA AYUELA, María Auxiliadora**
  **46115 Alfara del Patriarca - Valencia (ES)**

(74) Representative: **Temiño Ceniceros, Ignacio**
  **Abril Patentes y Marcas, S.L.**
  **Calle Zurbano, 76 - 7°**
  **28010 Madrid (ES)**

(54) **AMPHOTERICIN B FORMULATIONS FOR INHALATION BASED ON COLLAPSED MICROPARTICLES CONTAINING CARBOHYDRATES AND AMINO ACIDS**

(57)    Antifungal formulations for inhalation based on collapsed microparticles containing carbohydrates and amino acids.

Commercial antifungal formulations for the treatment and prophylaxis of pulmonary infections caused by fungi or intracellular pathogens (such as leishmaniasis or tuberculosis) are usually based on lipid excipients and, in most cases, their use is intended for intravenous or aerosol administration. However, a formulation that would allow their administration using dry powder inhalers would be more desirable.

The present invention describes new pharmaceutical formulations of antifungals in the form of collapsed microparticles which do not contain lipid excipients but mono and oligosaccharides as well as amino acids so that they can be administered using dry powder inhalers or by using aerosols.

## Description

Antifungal formulations for inhalation based on collapsed microparticles containing carbohydrates and amino acids.

## FIELD OF THE ART

The present invention falls within the technical field of the manufacture of pharmaceutical preparations. More specifically, it refers to the preparation of formulations of amphotericin B pharmaceutical compositions.

## BACKGROUND OF THE INVENTION

Amphotericin B (AmB) is the drug currently used as gold-standard in the treatment of pulmonary fungal infections and leishmaniasis due to its broad spectrum of activity and low resistance. Currently, amphotericin B is only marketed as a lyophilized powder for extemporaneous reconstitution before intravenous administration. However, its use is associated with adverse effects such as fever, chills, nausea and vomiting, and, more significantly, nephrotoxicity.

To reduce this toxicity, lipid formulations of amphotericin B have previously been proposed as an alternative to conventional treatment. These formulations allow a higher dosage and thus an increase in the therapeutic index.

In most cases, these lipid-based formulations are administered intravenously. Typically, these lipid formulations of AmB are marketed in lyophilized powder form for immediate reconstitution before intravenous administration (AmBisome® and Abelcet®, for example).

However, it is desirable to have a formulation of AmB that can be administered by the inhalation route, as it allows a rapid action of the drug and is useful, in particular, for the local administration acting on the lower respiratory tract.

Most clinical trials on AmB delivery are focused on the safety and efficacy of commercial inhaled AmB lipid formulations.

Inhaled medications can be delivered using metered-dose inhalers (known as pMDIs) such as aerosols and nebulizers; or dry powder inhalers (DPIs). Each of these requires a formulation strategy to ensure successful drug release into the lung. But, among all of them, DPIs have advantages such as consistency of the dose delivered to the airways, good deposition efficiency, ease of administration, and formulation stability. In most cases, their use is intended for aerosol administration.

One of the limitations of DPI formulations is that the inspiratory flow of the patient determines the efficacy of inhalation and deposition of the drug in the lungs (De Pablo, E, Fernández-García R, Ballesteros MP, Torrado JJ, Serrano D.R. Nebulised antibiotherapy: conventional versus nanotechnology-based approaches, is targeting at a nano-scale a difficult subject? Annals of Translational Medicine, 2017 5(22): p. 1-16.). Therefore, drugs may not be successfully administered in patients suffering from severe fungal or parasitic lung infections, with greatly diminished lung capacity, or patients with a lack of hand-breathing coordination. In this regard, a versatile DPI formulation that could be both administered as a DPI and easily reconstituted in water followed by nebulization would be of great advantage; especially for pediatric and geriatric populations or patients with difficulties in achieving deep inspiration (Vartiainen, V., et al., Pulmonary administration of a dry powder formulation of the antifibrotic drug tilorone reduces silica-induced lung fibrosis in mice. Int J Pharm, 2018. 544(1): p. 121-128). The latter strategy based on the reconstitution of the powder in water for nebulization could be useful in those patients requiring dose adjustment, for example, in the case of prophylactic treatments or severe infections (Rijnders, B.J., et al., Aerosolized liposomal amphotericin B for the prevention of invasive pulmonary aspergillosis during prolonged neutropenia: a randomized, placebo-controlled trial. Clin Infect Dis, 2008. 46(9): p. 1401-8; Paranjpe, M. and C.C. Muller-Goymann, Nanoparticle-mediated pulmonary drug delivery: a review. Int J Mol Sci, 2014. 15(4): p. 5852-73).

On the other hand, experience with inhaled antibiotics designed for parenteral administration shows that they can cause bronchial irritation due to the use of non-physiological excipients in their composition. Furthermore, there is not only a clinical need for DPI antifungals but also for DPI formulations combining antifungals and bronchodilators.

Therefore, a formulation of amphotericin B that can be administered in powder form and is free of excipients that cause bronchial irritation and can incorporate other antifungals and bronchodilators to accentuate its effect would be desirable.

## EXPLANATION OF THE INVENTION

The present invention describes pharmaceutical formulations of amphotericin B containing amphotericin B alone or in combination with other antifungal drugs to increase their efficacy or in combination with other bronchodilator drugs, for pulmonary administration in dry powder form. These formulations constitute a solution to the clinical problem of the lack of drugs available for the treatment and prophylaxis of pulmonary infections caused by fungi or intracellular pathogens such as leishmaniasis or tuberculosis.

More specifically, the invention concerns new pharmaceutical formulations of amphotericin B (AmB) for pulmonary administration, as well as their method of preparation and applications.

Based on clinical need, formulations adapted to the pulmonary route have been developed containing AmB alone or in combination with other antifungal drugs (such as itraconazole) to increase their efficacy, or in combination with bronchodilator drugs (such as salbutamol). These are formulations that can be administered either as a dry powder (which is much easier to administer without requiring coordination between breathing and pulsation) or as aerosols administered with a nebulizer after reconstitution in water. 15(4): p. 5852-73).

The first aspect of the invention concerns the development of collapsed microparticles without lipid excipients having a geometrical and aerodynamic size suitable for the pulmonary route (1 - 5 $\mu$m) and with an antifungal drug loading of more than 25% of the total content of the formulation, wherein the drug may be in amorphous or semicrystalline form (up to 50%).

Specifically, the method for the preparation of the formulations of the present invention comprises the following steps:

(a) An aqueous solution of $\gamma$--cyclodextrin is prepared and the pH is adjusted between 12 and 14.
b) AmB (in an amount between 5 - 40% of the total weight of the formulation) is then added to the solution of step (a) in a weight ratio AmB: $\gamma$--cyclodextrin between 1:1 and 1:50 and stirred constantly until complete dissolution.
c) The pH of the mixture of step (b) is adjusted to a value between 6 and 8 and is sonicated if necessary to obtain a homogeneous mixture.
d) A monosaccharide (e.g. mannose, raffinose, or mannitol) is added to the aqueous suspension in a proportion between 5 and 40% by weight of the total (AmB: monosaccharide weight ratio of 1:1).
e) An amino acid (such as leucine or isoleucine) is then incorporated into the above suspension in proportions below 20% by weight of the total formulation.

For the preparation of the dry powder formulation for inhalation, the following steps are also carried out:

(f) The aqueous suspension is atomized by spray drying. The air or nitrogen inlet flow can vary between 600 and 800 Uh, although preferably 742 Uh is used; the aqueous suspension is pumped at a rate between 2 and 6 ml/min, preferably at 2.5 ml/min; the evaporation temperature is maintained above 110°C, preferably 150°C; and the suction force above 90%.
g) After atomization, the atomized powder is collected from the collecting vessel.

A second aspect of the invention concerns the use of low molecular weight mono- and oligosaccharides as bioactive excipients for the vectorization of the antifungal drug towards the lung epithelium such as, for example, cyclodextrins and mannose.

Regarding oligosaccharides, it is known that cyclodextrin can diffuse through the mucus produced in certain pulmonary infections and facilitate the destabilization of the biofilm formed by microorganisms. These defects are usually temporary and reversible, minimizing its toxicity at the pulmonary level. The proportion of cyclodextrin used in the present invention for the solubilization of certain antifungals such as AmB is lower than that described previously (WO2012042072). A low ratio of cyclodextrins to AmB (e.g., 1:1 by weight) allows a balance to be established between solubilized and added AmB. This balance improves the benefit-risk balance at the pulmonary level.

It is also known that the combination of AmB with monosaccharides, such as mannose, has a double effect: on the one hand, it acts as a chemotactic agent attracting cellular pathogens and, on the other hand, it intervenes in the regulation of cellular immunity by being able to interact with receptors located on the surface of macrophages, activating the immune response and promoting the eradication of pathogenic organisms (Brown, G.D., Dectin-1: a signalling non-TLR pattern-recognition receptor. Nat Rev Immunol, 2006. 6(1): p. 33-43). Therefore, the formulations of the present invention may be useful for treatment against intracellular pathogens that are localized at the macrophage level, such as tuberculosis or leishmaniasis (Pinto, M.R., E. Barreto-Bergter, and C.P. Taborda, Glycoconjugates and polysaccharides of fungal cell wall and activation of immune system. Braz J Microbiol, 2008. 39(2): p. 195-208).

A third aspect of the invention concerns the combination of AmB with amino acids, such as leucine, in low proportions (< 20 %) as facilitators of particle flow at the respiratory tract level (Focaroli, S., et al., A Design of Experiment (DoE) approach to optimise spray drying process conditions for the production of trehalose/leucine formulations with application in pulmonary delivery. Int J Pharm, 2019. 562: p. 228-240; Molina, C., et al., Agglomerated novel spray-dried lactose-leucine tailored as a carrier to enhance the aerosolization performance of salbutamol sulfate from DPI formulations. Drug Deliv Transl Res, 2018. 8(6): p. 1769-1780), thus conferring to the particles a collapsed morphology with anti-adherent character allowing better lung deposition and lower susceptibility to moisture, prolonging their physicochemical stability (Mah, P.T., et al., The use of hydrophobic amino acids in protecting spray dried trehalose formulations against moisture-induced changes. Eur J Pharm Biopharm, 2019. 144: p. 139-153). In addition, the combination of mannose and leucine in the formulation has a protective ability against red blood cells, which is very important.

A fourth aspect of the invention concerns the development of highly versatile atomized pulmonary formulations, which can be administered both as dry powder inhalers and as aerosols with low susceptibility to the patient's inspiratory airflow. The collapsed microparticles show good lung deposition at different inspiratory airflows (between 60 and 30 L/min). Moreover, they can be reconstituted in water, resulting in an optimal size in suspension, and can be aerosolized while maintaining a correct deposition in the respiratory tract. Furthermore, in vivo clearance, after pulmonary administration has demonstrated longer times of the microparticles in the lung and, consequently, a better therapeutic effect.

A fifth aspect of the invention is the combination of AmB with other antifungal drugs or bronchodilators. Depending on the excipients used and the conditions employed for their manufacture, the microparticles can contain more than one antifungal drug, such as itraconazole, which can increase their efficacy. It has been possible to load up to 40% of drugs into the microparticles while maintaining a good in vitro lung deposition profile. Similarly, the combination with bronchodilator drugs, such as salbutamol, is also feasible, maintaining good particle deposition in the lung.

**BRIEF DESCRIPTION OF THE DRAWINGS**

To complement the description that is being made to help a better understanding of the characteristics of the invention, a set of drawings is attached as an integral part of the description, in which the following has been represented for illustrative and non-limiting purposes:

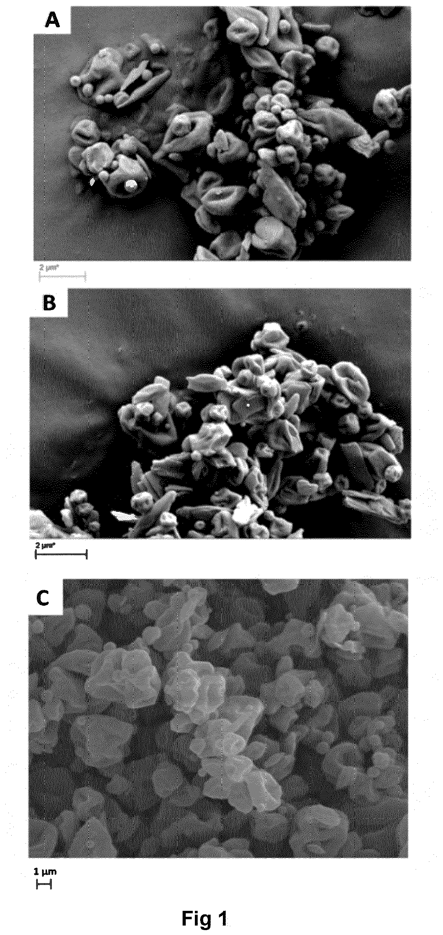

**Figure 1.** Physicochemical characterization of the collapsed microparticles of AmB and Itraconazole: (a) SEM of the semi-crystalline formulation of AmB, (b) SEM of the amorphous formulation of AmB and (c) SEM of the itraconazole formulation.

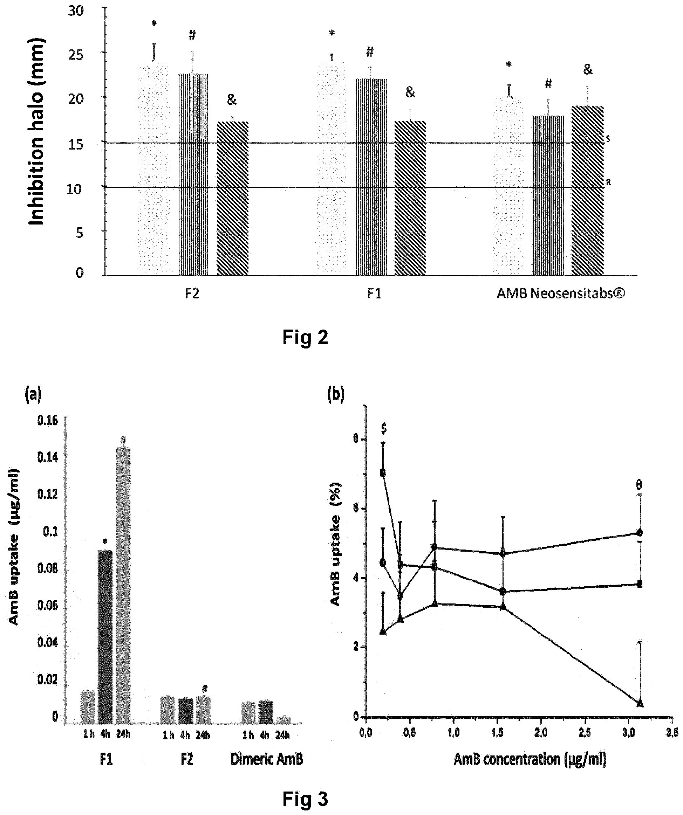

**Figure 2.** *In vitro* antifungal activity against *Candida ssp.* Key: Significant statistical differences (p<0.05) in *C. albicans, C. parasilopsis and C. glabrata* are expressed by *, # and &, respectively, compared to commercial Neosensitab® discs of AmB.

**Figure 3.** Study of macrophage uptake: (a) uptake of AmB microparticles in the presence of macrophages using an AmB concentration of 1.56 μg/ml after 1, 4 and 24 h of exposure. (b) uptake of AmB microparticles in the presence of macrophages at different AmB concentrations after 1h of exposure. Key: ■ semicrystalline AmB microparticles; ● amorphous AmB microparticles; ▲dimeric AmB.

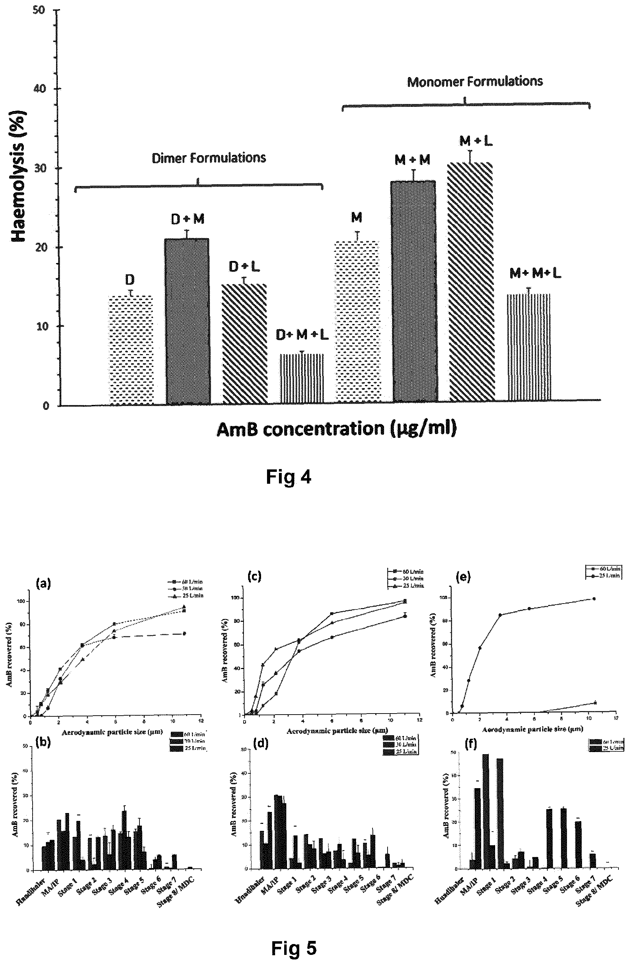

**Figure 4.** Hemolytic toxicity of the excipients used in the invention of collapsed microparticles against AmB in various aggregation states: dimeric (at a concentration of 62.5 μg/ml) and monomeric (at a concentration of 25 μg/ml). Key: dimeric AmB (D); dimeric AmB + mannose (D+M); dimeric AmB + leucine (D+L); dimeric AmB + leucine + mannose (D+L+M); monomeric AmB (M); monomeric AmB + mannose (M+M); monomeric AmB + leucine (M+L); monomeric AmB + mannose + leucine (M+M+L).

**Figure 5.** Aerodynamic size (MMDA) and particle fraction (FPF) of AmB formulations under three different conditions: (i) dry powder formulation (25 mg) for patients with normal inspiratory capacity (60 L/min for 4 s); (ii) dry powder formulation (25 mg) for patients with reduced inspiratory capacity (30 L/min for 8 s) and (iii) dry powder formulation (25 mg) reconstituted with water (5 ml) to a concentration of 5 mg/ml and nebulized with a mesh and ultrasound nebulizer device at 25 L/min for 15 min. Key; a) Cumulative AmB deposition as a function of particle size and b) The percentage of AmB recovered in different states from the inhaler to lower parts of the lungs for semicrystalline AmB formulation. c) Cumulative AmB deposition as a function of particle size and d) the percentage of AmB recovered in different states from the inhaler to lower parts of the lungs for amorphous AmB formulation. e) Cumulative AmB deposition as a function of particle size and f) the percentage of AmB recovered in different states from the inhaler to lower parts of the lungs for AmBisome® (commercial formulation).

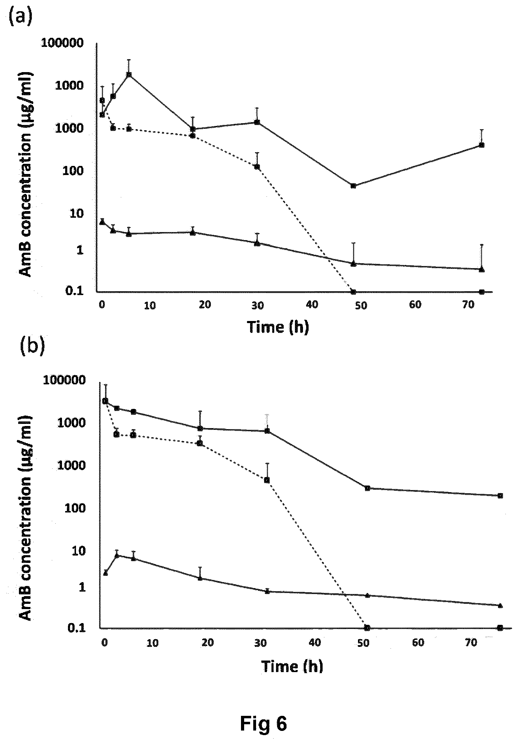

**Figure 6.** Pharmacokinetic profile after intratracheal administration of 5 mg/kg of the semi-crystalline dry powder AmB formulation F1 (a) and the amorphous powder AmB formulation F2 (b), compared to the commercial formulation AmBiosme®. Key: -- ■ -- AmBisome® concentration in plasma after intravenous administration, - ▲ - AmB concentration in lung epithelial tissue, and - ■ - AmB concentration in plasma.

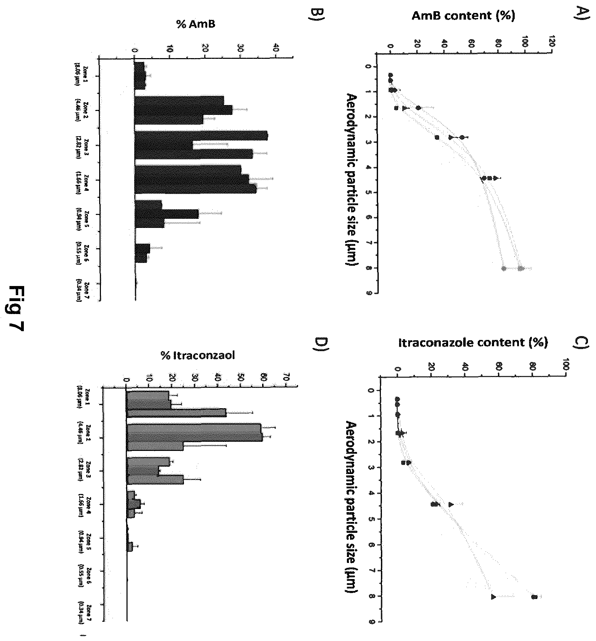

**Figure 7.** AmB formulations combined with itraconazole. Aerodynamic particle size (MMDA) and particle fraction (FPF) below 5 and 3 μm. Dry powder formulation (25 mg) for patients with normal inspiratory capacity (60 L/min for 4 s). Key: - ■ -F3 (20% total antifungals), - ● - F4 (30% total antifungals) and - A - F5 (40% total antifungals). A and B) Deposited amount of AmB in the different impaction layers and C and D) Deposited amount of itraconazole in the different impaction layers.

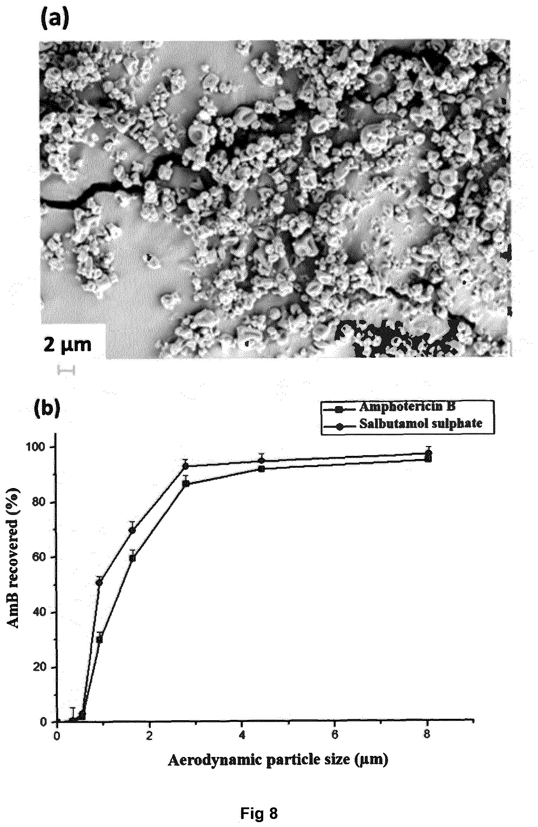

**Figure 8.** Combined formulation of AmB with salbutamol. Key: A) SEM of the collapsed microparticles containing both drugs. B) In vitro deposition at 60 L/min for 4 s using a next generation impactor (NGI).

A list of the various elements depicted in the figures that are integrated in the invention is provided below:

F1: Crystalline formulation (45% crystallinity) of AmB containing a 1:1:1:1 ratio of AmB: $\gamma$-cyclodextrin:mannose by weight and 10.4% leucine.

F2: Amorphous AmB formulation containing a ratio of AmB: $\gamma$-cyclodextrin:mannose by weight 1:1:0.2 and 12.5 % leucine.

F3: AmB formulation containing 20% antifungals.

F4: AmB formulation containing 30% antifungals.

F5: AmB formulation containing 40% antifungals.

## PREFERRED EMBODIMENTS OF THE INVENTION

The present invention is illustrated by the following examples, which are not intended to be limiting in scope.

**Example 1.**

This example relates to the preparation of AmB formulations following the method described in the invention.
An aqueous solution of $\gamma$-cyclodextrin is prepared and the pH of this solution is adjusted to between 12 and 14. AmB (in an amount equivalent in weight to that of the cyclodextrin (1:1 ratio) is then added and stirred constantly until complete dispersion. The pH of the mixture of step (b) is adjusted to a value between 6 and 8 and sonicated for 10 min in a water bath. Mannose is added to the aqueous suspension in a ratio AmB: $\gamma$-cyclodextrin:mannose of 1:1:1 (by weight) for the formulation described as F1, or AmB: $\gamma$-cyclodextrin:mannose in a ratio of 1:1:0.2 (by weight) for the formulation described as F2. An amino acid (such as leucine) is then incorporated into the above suspension in proportions below 20% by weight of the total formulation, in particular 10.4% for F1 and 12.5% for F2.
The obtained suspension is atomized in a Mini Büchi.B191 (Büchi Labortechnik AG, Switzerland) using a high-efficiency cyclone, setting the inlet temperature at 130 - 170°C, the spray flow rate at 2 - 6 mL/min (spray rate 5-15%), the nitrogen flow rate at 500 - 800 NL/h and the aspirator force at 85 - 100 %. After the atomization of the solution, the particles are collected in a collector.

**Example 2.**

This example concerns the preparation of itraconazole formulations following the described method.
In the itraconazole formulations, several aqueous suspensions were prepared with the same total solids concentration of 0.5% and a total amount of drug between 10-30%. For the formulation with a total of 20% drug, 200 mg of $\gamma$-cyclodextrin was dissolved in 200 ml of deionized water. 200 mg itraconazole, 200 mg L-leucine and 400 mg mannitol were added to the suspension and sonicated to obtain a homogeneous suspension before being atomized.
In Figure 1c, itraconazole microparticles with similar morphology to those of AmB are observed.

**Example 3.**

This example shows the influence of the atomization variables of the formulations of Example 1.
All prepared formulations had a particle size between 1 and 5 $\mu$m, the smaller the particle size, the higher the nitrogen flow rate. The suction force and inlet temperature have the opposite effect on the yield so higher yields are obtained at lower suction force and high inlet temperature.

**Example 4.**

In this example, the influence of the ratios of AmB, excipients, and amino acids on the properties of the prepared AmB formulation is shown.

Formulations are prepared by varying the AmB: γ-cyclodextrin weight ratio between 1:1 and 1:100 by weight, AmB:mannose weight ratio between 1:1 and 1:5 by weight and leucine used to improve flow properties between 5 and 15% by weight.

The parameters of the atomization process are maintained at a 10% atomization rate (4 mL/min), nitrogen flow rate of 742 Nl/h, inlet temperature 150°C, and suction rate of 90%.

In relation to the degree of crystallinity, the most influential variables are the AmB ratio: γ-cyclodextrin and the AmB:mannose ratio. The higher the degree of crystallinity, the higher the amount of mannose used and the lower the amount of γ-cyclodextrin and leucine. Figure 1 shows the physicochemical characterization of two formulations with different degrees of crystallinity, F1 (45% crystallinity) and F2 (amorphous). The ratio AmB: γ-cyclodextrin:mannose is 1:1:1 (by weight) and the percentage of leucine is 10.4% for F1; the ratio AmB: γ-cyclodextrin:mannose is 1:1:0.2 (by weight) and the percentage of leucine is 12.5% in F2.

In the micrographs obtained by SEM for F1 and F2 (Figure 1a-b, respectively), hemispherical collapsed particles with rough surfaces are observed. Small non-encapsulated crystals of size close to 1 μm are also observed in F1.

## Example 5.

This example shows the effect of the type of monosaccharide on the collapsed microparticles obtained.

The substitution of mannose for raffinose resulted in microparticles with a larger geometrical size (5-10 μm).

## Example 6.

In this example the *in vitro* antifungal activity of AmB formulations is tested.

The antifungal activity of atomized AmB formulations described in the previous examples is tested against three different strains of *Candida spp. (Candida spp., Candida albicans; CECT 1394, Candida Glabrata 60750 and Candida Parassilopsis 57744)*. The antifungal activity is tested by agar diffusion assay (Ruiz, H.K. et al. Int, J. Pharm, 2014. 473(1-2): p. 148-57).

Inhibition zone diameters are measured at points where there is complete inhibition of yeast growth. Isolates are classified as AmB susceptible (S) when the zone of inhibition is greater than 15 mm, resistant (R) if the zone of inhibition is less than 10 mm and intermediate (I) if the zone of inhibition is between 11 and 14 mm. Figure 2 shows that the three *Candida* strains tested are susceptible to formulations F1 and F2 with an inhibition halo greater than 15 mm. F1 and F2 show significantly higher efficacy ($p < 0.05$) than the commercial Neo-Sansitabs® AmB discs against *C. albicans* and *C. parapsilosis.*

## Example 7.

In this example, macrophage uptake of the AmB from the formulations of the invention is studied.

J774 (murine monocyte-like cell line) cells are grown in MEM (Minimum Essential Medium) developed by Eagle in the presence of FBS (Fetal Bovine Serum), 100 IU/mL penicillin G and 100 μg/mL streptomycin in 25 mL flask at 37°C and humidified 5% $CO_2$/air atmosphere. J774 cells (100 μL equivalent to 5x105 cells/mL) are incubated at 37°C with 100 μL of each AmB formulation before being reconstituted with deionized water and diluted to various concentrations, ranging from 25 to 0.195 μg/mL). Dimeric AmB is used as a control at the same concentrations. After various incubation times (1, 4 and 24 h), the supernatant is removed and the entire plate is washed with cold macrophage medium (RPMI). Cell lysis is carried out with 250 μl of 1% Triton in PBS followed by 30 min under mild agitation. Then, 100 μl is transferred to another 96-well plate and 100 μl of methanol is added to solubilize the AmB and precipitate the cell debris. The plate is centrifuged for 10 minutes at 2500 rpm. Finally, the supernatant is collected and analyzed by HPLC.

Formulations F1 and F2 differ in the degree of crystallinity of the AmB present in the microparticles and contain AmB:CD:mannose in 1:1:1 and 1:1:0.2 ratios, respectively, and leucine in ratios close to 10%. To study the influence of the excipients used in the claimed formulations (CD, mannose and leucine), F1 and F2 (containing mannose and leucine) are compared with aqueous AmB formulations containing only sodium deoxycholate (DOC) or CD. AmB dissolved in water and depending on the excipients, can be found in different aggregate states. In this case, AmB in DOC (AmB:DOC ratio 50:41 by weight) is in a dimeric state and AmB in CD (AmB:CD ratio 1:100 by weight) is in a monomeric state. Therefore, these formulations are referred to as dimeric AmB and monomeric AmB.

The in vitro uptake profile (Figure 3) of the atomized AmB formulations in differentiated J77A cells is markedly different from that of dimeric AmB. Formulation F1 shows 5- to 8-fold higher uptake than F2 at 4 and 24 h ($p < 0.05$) and 5- to 14-fold higher uptake than dimeric AmB at the same times ($p < 0.05$). The uptake of the F2 formulation is similar to dimeric AmB except at long times (24h) (Figure 3a).

No statistical differences are observed at 1 h between the three formulations when tested at intermediate concentrations (0.4 - 1.56 μg/ml). However, clear differences are seen at low (0.2 μg/ml) and high (3.125 μg/ml) concentrations. F1 shows faster uptake than F2 and dimeric AmB at low concentrations, which may be related to its larger particle size in solution (20 times larger), making the F1 formulation more attractive for macrophage uptake. Dimeric AmB shows a decrease in uptake at high concentrations, which may be a result of saturation. However, F1 and F2, which contain mannose that can bind to receptors located on the macrophage surface, are uptaken by macrophages even at higher concentrations (Figure 3b).

**Example 8.**

In this example, the hemolytic effect of excipients of atomized AmB formulations is shown.

Blood from healthy volunteers contained in EDTA-coated Vacutainer tubes is centrifuged at 1000 rpm for 5 minutes and plasma and hematocrit levels are marked in the tube. The supernatant is discarded from the tube and the tube is filled to the marked plasma level with 150 mM NaCl solution and mixed. The tube is centrifuged again (5 minutes at 1000 rpm) and the supernatant is discarded again. The red blood cells (RBC) are washed twice with 150 mM NaCl solution. The supernatant is then discarded and the RBCs are diluted with PBS (phosphate-buffered saline) at pH 7.4 to a final concentration of 4% ($4.81 \times 10^5$ RBCs/ml). The diluted RBCs are added to 96-well plates (180 μl/well).

To compare the effect of leucine and mannose on different aggregation states of AmB, dimeric and monomeric dilutions of AmB formulations are prepared as described in Example 7, and 20 μl of each is placed in the wells. For positive control, 20 μl of Triton X-100 solution is also added to a well. For negative control, 20 μl of PBS at pH = 7.4 is added. The plates are incubated at 37°C for 1 hour. Then, they are centrifuged at 2500 rpm for 5 minutes to sediment erythrocytes. The supernatants (50 μl) are transferred to a transparent 96-well flat-bottom plate. The absorbance (ABS) of the supernatants is measured using a plate reader (BioTek, ELx808) at 595 nm. The percentage of hemolysis is calculated using the equation:

$$\text{Hemolysis (\%)} = \frac{(\text{ABS}_{\text{sample}} - \text{ABS}_{\text{PBS}})}{(\text{ABS}_{\text{Triton}} - \text{ABS}_{\text{PBS}})}$$

Figure 4 shows the result of the study of the hemolytic toxicity of the excipients used in the invention on collapsed microparticles against AmB in various aggregation states: dimeric (at a concentration of 62.5 μg/ml) and monomeric (at a concentration of 25 μg/ml). It follows that the excipients (leucine and mannose) have a protective effect on RBCs only when used in 1:1 ratio by weight, but not separately, in the case of both dimeric and monomeric AmB.

**Example 9.**

Lung deposition of AmB formulations is shown in this example.

To determine the *in vitro* lung deposition of formulations F1 and F2, the aerodynamic particle size (MMDA) and particle fraction (FPF) below 5 and 3 μm are calculated according to the specifications of the European Pharmacopoeia 9.0 - Preparations by Inhalation for two different conditions: and 60 L/min for 4 seconds and 30 L/min for 8 seconds.

The formulations are also reconstituted in deionized water at 5 mg/ml to study lung deposition during nebulization for 15 minutes at 25 L/min. Tests are also performed with AmBisome® (intravenous lyophilized liposomal formulation) being incorporated into dry powder inhalation capsules or being reconstituted in deionized water at the same concentration.

Data for each of the compositions compared are shown in the table below (FPF, fine particle fraction, MMAD, Mass Median Aerodynamic Diameter).

| Conditions | F1 | | | F2 | | | AmBisome® | | |
|---|---|---|---|---|---|---|---|---|---|
| | FPF <5 μm (%) | FPF <3 μm (%) | MMAD (μm) | FPF <5 μm (%) | FPF <3 μm (%) | MMAD (μm) | FPF <5 μm (%) | FPF <3 μm (%) | MMAD (μm) |
| 60 L/min, 4 s | 81.45 | 67.16 | 1.66 | 79.32 | 53.27 | 2.42 | 4.26 | 0.43 | 4.46 |
| 30 L/min, 6 s | 68.95 | 52.47 | 3.48 | 53.98 | 42.12 | 3.28 | - | - | - |
| 25 L/min, 15 s | 63.84 | 46.06 | 3.85 | 55.11 | 47.13 | 4.14 | 95.80 | 74.38 | 2.50 |

As seen in Figure 5, atomized formulations F1 and F2 show good lung deposition at 60 L/min with FPF < 5 μm close to 80% and MMAD below 3 μm. When comparing deposition at different airflow rates, F1 shows a more consistent profile, while F2 is more sensitive to changes in airflow rate, exhibiting a significant 25% reduction in FPF < 5 μm at 25 and 30 L/min. In addition, F2 exhibits greater mass loss in the inhaler and higher parts of the respiratory tract. The commercial formulation AmBisome® shows a very different deposition profile between 60 L/min from its release from a capsule in a dry powder inhaler and nebulized after being reconstituted in deionized water and nebulized at 25 L/min.

Collapsed microparticles of F1 and F2 in the solid state showed good lung deposition at different inspiratory airflows (between 60 and 30 L/min). Moreover, they can be reconstituted in water, resulting in an optimal size in suspension, and can be aerosolized while maintaining good deposition in the respiratory tract.

**Example 10.**

This example refers to the pharmacokinetic profile of AmB formulations.

The pharmacokinetic profile of the formulations of the present invention is determined using OFA 250/275 g rats. The first group (G1) was administered 5 mg/kg of AmBisome® intravenously; groups G2 and G3 were administered formulations F1 and F2 in doses of 5 mg/kg intratracheally. Figure 6 shows higher concentrations of F1 than F2 after prolonged periods, which may be attributed to its semi-crystalline nature and larger particle size in solution. On the contrary, due to its amorphous nature, F2 is more soluble in water being more permeable through the pulmonary epithelial cells, and is eliminated faster from the organism. In both formulations, plasma concentrations above 1 μg/ml are maintained for at least 24 h; this indicates that a single daily administration would be sufficient to provide a prophylactic or therapeutic effect against Aspergillus spp.

However, plasma AmB levels drop very rapidly after intravenous administration of AmBisome® (below 1 μg/ml at 4 hours), which explains the low efficiency of this formulation when administered parenterally in critically ill patients (Figure 6).

**Example 11.**

This example shows the combination of the AmB formulations of the present invention with other antifungal drugs or bronchodilators.

Depending on the excipients used and the conditions employed for their manufacture, the microparticles may contain more than one antifungal drug, such as itraconazole and other monosaccharides such as mannitol. Thus, Figure 7 shows the good lung deposition profile of AmB microparticles loaded up to 40% with itraconazole.

Figure 8 shows that the incorporation of salbutamol makes the AmB particles more spherical with rough surfaces maintaining good particle deposition at the pulmonary level.

**Claims**

1. Amphotericin B formulation based on collapsed microparticles to be administered by inhalation in the form of dry powder, or aerosols after reconstitution in water, comprising amino acids, oligosaccharides, and monosaccharides as excipients, **characterized by** not containing lipid excipients and the microparticles having a geometric and aerodynamic size suitable for the pulmonary route (1 - 5 μm) with an antifungal drug load of more than 25% by weight of the dry powder of the total content of the formulation and where the drug can be in amorphous or semi-crystalline form (up to 50%).

2. Formulation according to claim 1, wherein the oligosaccharides and monosaccharides are γ-cyclodextrin and mannose, and the amino acid is leucine.

3. Formulation, according to claim 2, wherein the ratio AmB: mannose is 1:1 and leucine represents 10 - 13 wt.% of the total formulation.

4. Formulation, according to claim 3, wherein the ratio AmB: γ-cyclodextrin:mannose is 1:1:1.

5. Formulation, according to claim 3, further comprising other antifungal and/or bronchodilator drugs.

6. Formulation according to claim 5, wherein the added antifungal is itraconazole.

7. Formulation, according to claim 6, wherein the itraconazole is in a proportion of up to 40% within the microparticles.

8.  Formulation, according to claim 5, wherein the bronchodilator is salbutamol.

9.  Method for the preparation of the claimed formulation comprising the following steps:

    (a) Preparation of an aqueous solution of $\gamma$-cyclodextrin and adjusted pH between 12 and 14.
    b) Amphotericin B addition (in an amount between 5 - 40% of the total weight of the formulation) to the solution of step (a) in a weight ratio AmB: $\gamma$-cyclodextrin between 1:1 under constant stirring until complete dispersion.
    c) Adjustment of the pH of the mixture of step (b) to a value between 6 and 8, follow by sonication if necessary to obtain a homogeneous mixture.
    d) Addition of a monosaccharide to the aqueous suspension in a relation weight AmB: monosaccharide of 1:1.
    e) Addition of an amino acid to the previous suspension in proportions between 10 and 13% by total weight formulation.
    f) Atomization of the aqueous suspension by spray drying for the preparation of the dry powder formulation.
    g) Collection of the atomized powder from the collection vessel.

10. Method according to claim 9, wherein the antifungal is amphotericin B (AmB) the monosaccharide is mannose, and the amino acid is leucine.

11. Method, according to claim 9, wherein the atomization stage is carried out employing an air or nitrogen inlet flow rate between 600 and 800 Uh, the aqueous suspension is pumped at a rate between 2 and 6 ml/min, the evaporation temperature is maintained above 110°C, and the suction force above 80%.

12. Method according to claim 11, wherein the air or nitrogen flow rate is maintained at 742 L/h, the suspension is pumped at 2.5 ml/min, the evaporation temperature is maintained at 150°C, and the suction force is above 90%.

13. Use of the claimed formulation for the manufacture of an antifungal drug for inhalation administration.

14. Use according to claim 13, wherein the administration is by a dry powder inhaler (DPI).

15. Use according to claim 14, wherein the administration is by using aerosols, after reconstitution of the powder in water.

**Fig 1**

**Fig 2**

**Fig 3**

Fig 4

Fig 5

(a)

(b)

**Fig 6**

Fig 7

Fig 8

# INTERNATIONAL SEARCH REPORT

| | |
|---|---|
| International application No. | |
| PCT/ES2021/070885 | |

**A. CLASSIFICATION OF SUBJECT MATTER**

**See extra sheet**

According to International Patent Classification (IPC) or to both national classification and IPC

**B. FIELDS SEARCHED**

Minimum documentation searched (classification system followed by classification symbols)
A61K, A61P

Documentation searched other than minimum documentation to the extent that such documents are included in the fields searched

Electronic data base consulted during the international search (name of data base and, where practicable, search terms used)

EPODOC, INVENES, WPI, MEDLINE, NPL, EMBASE, BIOSIS

**C. DOCUMENTS CONSIDERED TO BE RELEVANT**

| Category* | Citation of document, with indication, where appropriate, of the relevant passages | Relevant to claim No. |
|---|---|---|
| X | WO 2007041090 A2 (NEKTAR THERAPEUTICS) 12/04/2007, Paragraphs [1 - 79]; paragraphs [135 - 262]; claims 1-53; | 1-3, 5-15 |
| X | WO 2007092088 A1 (NEKTAR THERAPEUTICS) 16/08/2007, the whole document | 1-3, 5-15 |
| X | CHENG S. N. et al. A Critical Review on Emerging Trends in Dry Powder Inhaler Formulation for the Treatment of Pulmonary Aspergillosis. Pharmaceutics, 28/11/2020, Vol. 12, pages 1161, | 1-3, 5-8, 13-15 |
| Y | <DOI: 10.3390/pharmaceutics12121161> the whole document | 9-12 |

☒ Further documents are listed in the continuation of Box C.       ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
|---|---|
| 31/03/2022 | **(01/04/2022)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | M. Cumbreño Galindo Telephone No. 91 3496880 |

Form PCT/ISA/210 (second sheet) (January 2015)

## INTERNATIONAL SEARCH REPORT

| International application No. |
| --- |
| PCT/ES2021/070885 |

| C (continuation). | DOCUMENTS CONSIDERED TO BE RELEVANT | |
| --- | --- | --- |
| Category * | Citation of documents, with indication, where appropriate, of the relevant passages | Relevant to claim No. |
| Y | WO 9632096 A1 (INHALE THERAPEUTIC SYST) 17/10/1996, pages 1 - 20; pages 28 - 42; | 9-12 |
| A | CN 110801433B B (UNIV JIANGNAN) 18/02/2020, the whole document | 1-15 |
| A | MEHENNI L. et al. Preparation and Characterization of Spherical Amorphous Solid Dispersion with Amphotericin B. Pharmaceutics, 16/11/2018, Vol. 10, pages 235, <DOI: 10.3390/pharmaceutics10040235> the whole document | 1-15 |
| A | MERCHANT Z. I. Delivery of nanocarrier-loaded hydrophobic drugs via the airways. Doctoral Thesis, University College London, School of Pharmacy Department of Pharmaceutics, 2017 pages 62 - 123; pages 237 - 240; | 1-15 |
| A | MEHTA P. et al. Imagine the Superiority of Dry Powder Inhalers from Carrier Engineering. Journal of Drug Delivery, 14/01/2018, Vol. 2018, N° Article ID 5635010, <DOI: https://doi.org/10.1155/2018/5635010> the whole document | 1-15 |

Form PCT/ISA/210 (continuation of second sheet) (January 2015)

## INTERNATIONAL SEARCH REPORT

International application No.

PCT/ES2021/070885

**CLASSIFICATION OF SUBJECT MATTER**

*A61K31/7048* (2006.01)
*A61K47/40* (2006.01)
*A61K47/26* (2006.01)
*A61K47/18* (2017.01)
*A61K9/72* (2006.01)
*A61P31/10* (2006.01)

Form PCT/ISA/210 (extra sheet) (January 2015)

## INTERNATIONAL SEARCH REPORT

Information on patent family members

International application No.

PCT/ES2021/070885

| Patent document cited in the search report | Publication date | Patent family member(s) | Publication date |
|---|---|---|---|
| WO2007041090 A2 | 12.04.2007 | US2009032427 A1 | 05.02.2009 |
| | | EP1928525 A2 | 11.06.2008 |
| | | CA2623256 A1 | 12.04.2007 |
| | | AU2006297394 A1 | 12.04.2007 |
| | | AU2006297394B B2 | 12.09.2013 |
| WO2007092088 A1 | 16.08.2007 | US2012128728 A1 | 24.05.2012 |
| WO9632096 A1 | 17.10.1996 | KR19980703876 A | 05.12.1998 |
| | | US6136346 A | 24.10.2000 |
| | | US6187344 B1 | 13.02.2001 |
| | | US6358530 B1 | 19.03.2002 |
| | | US6582729 B1 | 24.06.2003 |
| | | MX9707890 A | 31.03.1998 |
| | | JPH11501657 A | 09.02.1999 |
| | | JP3708553B B2 | 19.10.2005 |
| | | ES2237767T T3 | 01.08.2005 |
| | | EP0820277 A1 | 28.01.1998 |
| | | EP0820277 A4 | 30.05.2001 |
| | | DE69634246T T2 | 12.01.2006 |
| | | CA2218074 A1 | 17.10.1996 |
| | | CA2218074 C | 08.10.2002 |
| | | AU5550396 A | 30.10.1996 |
| | | AU706195B B2 | 10.06.1999 |
| | | AT287703T T | 15.02.2005 |
| CN110801433 A | 18.02.2020 | CN110801433B B | 04.05.2021 |

Form PCT/ISA/210 (patent family annex) (January 2015)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012042072 A

**Non-patent literature cited in the description**

- **DE PABLO, E ; FERNÁNDEZ-GARCÍA R ; BALLESTEROS MP ; TORRADO JJ ; SERRANO D.R.** Nebulised antibiotherapy: conventional versus nanotechnology-based approaches, is targeting at a nano-scale a difficult subject?. *Annals of Translational Medicine,* 2017, vol. 5 (22), 1-16
- **VARTIAINEN, V. et al.** Pulmonary administration of a dry powder formulation of the antifibrotic drug tilorone reduces silica-induced lung fibrosis in mice. *Int J Pharm,* 2018, vol. 544 (1), 121-128
- **RIJNDERS, B.J. et al.** Aerosolized liposomal amphotericin B for the prevention of invasive pulmonary aspergillosis during prolonged neutropenia: a randomized, placebo-controlled trial. *Clin Infect Dis,* 2008, vol. 46 (9), 1401-8
- **PARANJPE, M. ; C.C. MULLER-GOYMANN.** Nanoparticle-mediated pulmonary drug delivery: a review. *Int J Mol Sci,* 2014, vol. 15 (4), 5852-73
- **BROWN, G.D.** Dectin-1: a signalling non-TLR pattern-recognition receptor. *Nat Rev Immunol,* 2006, vol. 6 (1), 33-43
- **PINTO, M.R. ; E. BARRETO-BERGTER ; C.P. TABORDA.** Glycoconjugates and polysaccharides of fungal cell wall and activation of immune system. *Braz J Microbiol,* 2008, vol. 39 (2), 195-208
- **FOCAROLI, S. et al.** A Design of Experiment (DoE) approach to optimise spray drying process conditions for the production of trehalose/leucine formulations with application in pulmonary delivery. *Int J Pharm,* 2019, vol. 562, 228-240
- **MOLINA, C. et al.** Agglomerated novel spray-dried lactose-leucine tailored as a carrier to enhance the aerosolization performance of salbutamol sulfate from DPI formulations. *Drug Deliv Transl Res,* 2018, vol. 8 (6), 1769-1780
- **MAH, P.T. et al.** The use of hydrophobic amino acids in protecting spray dried trehalose formulations against moisture-induced changes. *Eur J Pharm Biopharm,* 2019, vol. 144, 139-153
- **RUIZ, H.K. et al.** *Int, J. Pharm,* 2014, vol. 473 (1-2), 148-57